Europäisches Patentamt

European Patent Office

Office européen des brevets

(11) Veröffentlichungsnummer: **0 387 858**
**A1**

## (12) EUROPÄISCHE PATENTANMELDUNG

(21) Anmeldenummer: **90104851.2**

(22) Anmeldetag: **14.03.90**

(51) Int. Cl.5: **A61B 17/22**

(30) Priorität: **14.03.89 DE 3908274**

(43) Veröffentlichungstag der Anmeldung:
**19.09.90 Patentblatt 90/38**

(84) Benannte Vertragsstaaten:
**AT BE CH DE DK ES FR GB GR IT LI LU NL SE**

(71) Anmelder: **STORZ MEDICAL AG**
**Unterseestrasse 47**
**CH-8280 Kreuzlingen(CH)**

(72) Erfinder: **Marlinghaus, Ernst H., Dr.**
**Rigistrasse 9**
**CH-8598 Bottighofen(CH)**

(74) Vertreter: **Münich, Wilhelm, Dr. et al**
**Kanzlei Münich, Steinmann, Schiller**
**Willibaldstrasse 36/38**
**D-8000 München 21(DE)**

(54) **Vorrichtung zur Erzeugung von fokussierten akustischen Wellenfeldern.**

(57) Beschrieben wird eine Vorrichtung zur Erzeugung von fokussierten akustischen Wellenfeldern für therapeutische Anwendungen und insbesondere zur Zerstörung von Konkrementen, Körpersteinen etc. mit einer Schallerzeugungseinheit, die in einem Koppelmedium ein Wellenfeld erzeugt, das durch die Formgebung der Abstrahlfläche und/oder das Wellenfeld reflektierende und/oder beugende Elemente fokussiert wird.

Die erfindungsgemäße Vorrichtung zeichnet sich dadurch aus, daß zur Variation der Fokusgröße die Wellenlänge der abgestrahlten Schallwellen variierbar sind.

EP 0 387 858 A1

## Vorrichtung zur Erzeugung von fokussierten akustischen Wellenfeldern

Die Erfindung bezieht sich auf eine Vorrichtung zur Erzeugung von fokussierten akustischen Wellenfeldern für therapeutische Anwendungen und insbesondere zur Zerstörung von Konkrementen, Körpersteinen etc. mit einer Schallerzeugungseinheit, die in einem Koppelmedium ein Wellenfeld erzeugt, das durch die Formgebung der Abstrahlfläche und/oder das Wellenfeld reflektierende und/oder beugende Elemente fokussiert wird.

Eine Vorrichtung zur Erzeugung von fokussierten akustischen Wellenfeldern für therapeutische Anwendungen und insbesondere zur Zerstörung von Konkrementen, Körpersteinen etc., gemäß dem Oberbegriff des Patentanspruchs 1 ist beispielsweise aus den deutschen Offenlegungsschriften 32 41 026, 33 18 871, 34 25 992 oder 34 37 862 sowie aus der nicht vorveröffentlichten älteren Patentanmeldung P 38 35 318.0 bekannt.

Bei der aus der DE-OS 32 41 026 bekannten Vorrichtung wird als "Schallerzeugungseinheit" eine Funkenstrecke verwendet, deren Wellenfeld von einem Ellipsoid reflektiert und so fokussiert wird.

Die in den DE-OSen 33 19 871 und 34 25 992 beschriebenen Vorrichtungen verwenden als Schallerzeugungseinheit piezoelektrische Wandler, die auf der Innenseite einer Kugelkalotte angeordnet sind. Aufgrund der Formgebung der Abstrahlfläche entsteht ein fokussierten Wellenfeld.

Aus der nicht vorveröffentlichten Patentanmeldung P 38 35 318.0 derselben Anmelderin ist eine Schallerzeugungseinheit bekannt, die eine Zylindergeometrie (im allgemeinen Sinne) aufweist. Durch einen entsprechend ausgebildeten Reflektor wird das Wellenfeld der Schallerzeugungseinheit dann fokussiert.

Weiterhin ist es bekannt, ebene Schallerzeugungseinheiten, die auf elektromagnetischer Basis arbeiten, in "rohrähnlichen" und als Linsen wirkende Schall-Führungs- und Bündelungssystemen anzuordnen.

Die aus den vorveröffentlichten Druckschriften bekannten Vorrichtungen zur Erzeugung von fokussierten akustischen Wellenfeldern für therapeutische Anwendungen und insbesondere zur Zerstörung von Konkrementen, Körpersteinen etc. weisen eine feste Fokusgröße auf, die sich vom Anwender nicht verändern läßt; damit kann der Anwender die Fokusgröße nicht an die Steingröße, -lage oder -art anpassen:

Beispielsweise haben Vorrichtungen, bei denen auf der Innenseite einer Kugelkalotte piezoelektrische Schallwandler angeordnet sind, einen vergleichsweise kleinen Fokus, während Vorrichtungen mit ebenen Schallerzeugungseinheiten auf elektromagnetischer Basis einen vergleichsweise ausgedehnten "Fokus" haben.

Der Erfindung liegt die Aufgabe zugrunde, eine Vorrichtung zur Erzeugung von fokussierten akustischen Wellenfeldern für therapeutische Anwendungen und insbesondere zur Zerstörung von Konkrementen, Körpersteinen etc. anzugeben, die eine leichte Einstellung der Fokusgröße erlaubt.

Eine erfindungsgemäße Lösung dieser Aufgabe ist mit ihren Weiterbildungen in den Patentansprüchen gekennzeichnet.

Überraschenderweise kann die erfindungsgemäße Aufgabe dadurch gelöst werden, daß weiterhin von einer Vorrichtung gemäß dem Oberbegriff des Patentanspruchs 1, also von einer Vorrichtung mit einer Schallerzeugungseinheit, die in einem Koppelmedium ein Wellenfeld erzeugt, das durch die Formgebung der Abstrahlfläche und/oder das Wellenfeld reflektierende und/oder beugende Elemente fokussiert wird, ausgegangen wird.

Der Erfindung geht dabei von dem Grundgedanken aus, daß bei einer Vorrichtung gemäß dem Oberbegriff des Anspruchs 1 die Fokussierung des Wellenfeldes "beugungsbegrenzt" ist. Damit ist gemeint, daß der Einfluß der Wellenlänge des Wellenfeldes, das durch die Formgebung der Abstrahlfläche und/oder reflektierende und/oder beugende Elemente "abgebildet" und insbesondere fokussiert wird, vergleichsweise groß ist.

Anders ausgedrückt, kann die Größe des Fokus in gewissen Grenzen bei ansonsten gleichen Geometriedaten der das Wellenfeld beeinflußenden Elemente dadurch variiert werden, daß die Wellenlänge des abgestrahlten Wellenfeldes variiert wird. Die in technischem Maßstab problemlos darstellbaren Wellenlängen-Änderungen sind dabei hinreichend, um die Fokusgröße an medizinische Forderungen anzupassen, die sich beispielsweise durch die Steingröße, -art und/oder -lage ergeben.

Weiterbildungen der Erfindung sind in den Unteransprüchen angegeben.

In den Ansprüche 2 bis 4 sind verschiedenen Maßnahmen angegeben, mit denen bei einer Vorrichtung, bei der die Schallerzeugungseinheit mittels einer Entladeeinheit ansteuerbar ist, die eine Kondensator-Bank zur Energiespeicherung aufweist, die Wellenlänge des erzeugten Wellenfeldes ohne großen Aufwand variierbar ist:

Gemäß Anspruch 2 sind zur Variation der Wellenlänge die einzelnen Kondensatoren der Kondensator-Bank wahlweise parallel und/oder in Reihe schaltbar, während gemäß Anspruch 3 zur Variation der Wellenlänge lastseitig Kondensatoren und/oder Induktivitäten zuschaltbar sind.

Diese Maßnahmen können im Prinzip bei beliebigen Vorrichtungen, also auch bei Vorrichtungen mit piezoelektrischen Schallwandlern angewendet werden.

Im Anspruch 4 ist beansprucht, daß zur Variation der Frequenz lastseitig die Induktivität schaltbar sind. Diese Lösung ist insbesondere von Vorteil bei einer Vorrichtung gemäß der älteren Patentanmeldung P 38 35 318.0, bei der die Schallerzeugungseinheit als elektromagnetische Druckwellenerzeugungseinheit ausgebildet ist, die Zylindergeometrie im allgemeinen Sinne aufweist, und mittels einer Entladeeinheit ansteuerbar ist, die eine Kondensator-Bank zur Energiespeicherung aufweist, und deren Wellenfeld ein insbesondere als Paraboloid ausgebildeter Reflektor fokussiert. Bei einer derartigen Vorrichtung kann die Variation der Wellenlänge des Wellenfeldes beispielsweise wie folgt erzielt werden:

Durch die vergleichsweise große Oberfläche der Abstrahlfläche der Schallerzeugungseinheit ist es möglich, daß die Schallerzeugungseinheit mehrere ineinander gewickelte Spulen aufweist, die alternativ oder miteinander verschaltet vom Strom der Entladeeinheit durchflossen werden.

Dies bedeutet, daß durch einen externen Umschaltvorgang, der beispielsweise vom behandelnden Arzt oder von einer Steuereinheit ausgelöst werden kann, die einzelnen Spulen, die die Schallerzeugungseinheit aufweist, alternativ oder in einer (teilweisen) Reihen- und/oder Parallelschaltung von der Entladeeinheit angesteuert werden. Da die verschiedenen Spulen unterschiedliche Induktivitäten aufweisen, ändern sich die Parameter des von den Spulen und den Kondensatoren der Kondensator-Bank zur Energiespeicherung gebildeten Schwingkreises, und damit auch die Wellenlänge des erzeugten Druckwellenfeldes.

Die in den vorstehend besprochenen Ansprüchen angegebenen Maßnahmen haben den Vorteil, daß es durch sie möglich ist, während eines Behandlungsvorgangs die Fokusgröße an die Charakteristiken des zu zerstörenden Konkrements, das beispielsweise mittels einer Ortungseinheit erfaßt wird, anzupassen. Das Umschalten kann dabei mittels "Knopfdruck" durch den behandelnden Arzt, aber auch durch eine entsprechende Steuereinheit, an die beispielsweise die Signale der Ortungseinheit angelegt sind, erfolgen.

Selbstverständlich ist es aber auch möglich, die Vorrichtung zur Erzeugung von fokussierten akustischen Wellenfeldern für therapeutische Anwendungen und insbesondere zur Zerstörung von Konkrementen, Körpersteinen etc. derart auszugestalten, daß die Spule bzw. Spulen austauschbar sind. Dabei ist es bevorzugt, daß sich die austauschbaren Spulen hinsichtlich Durchmesser, Windungszahl und Membranmaterial unterscheiden (Anspruch 6).

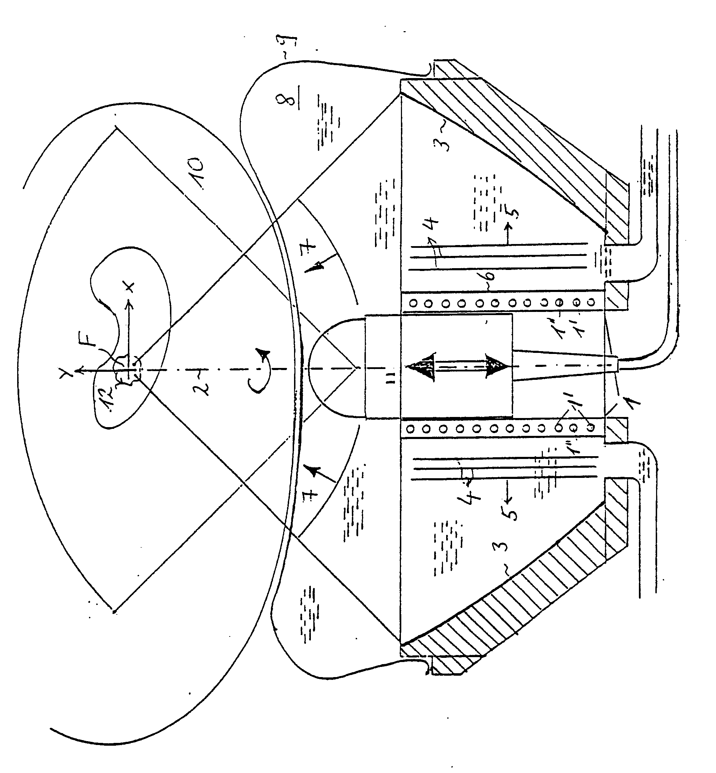

Die Erfindung wird nachstehend anhand eines Ausführungsbeispiels unter Bezugnahme auf die einzige Figur der Zeichnung näher beschrieben, die einen Querschnitt durch eine Vorrichtung zur Erzeugung von fokussierten akustischen Wellenfeldern zeigt.

Die dagestellte Vorrichtung zur Erzeugung von fokussierten akustischen Wellenfeldern ist in der nicht vorveröffentlichten Anmeldung P 38 35 318.0 näher beschrieben und weist eine Schallerzeugungseinheit 1 für die Therapiewelle auf, deren Abstrahlfläche 6 die Form eines Zylinders hat. Koaxial zur Zylinderachse 2 der Abstrahlfläche 6 der Schallerzeugungseinheit 1 ist ein rotationssymmetrischer Reflektor 3 angeordnet, dessen Innenfläche in jedem Schnitt die Form einer Parabel hat.

Da die Schallerzeugungseinheit zylindrische Wellenfronten 4 erzeugt, die sich in Radialrichtung 5 von der Quelle 1 wegbewegen, ergeben sich nach Reflexion an dem Reflektor 3 mit paraboloider Innenkontur fokussierte Wellenfelder 7, die im Fokuspunkt F zusammenlaufen und dort Leistungsdichten erzeugen, die therapeutisch wirksam sind.

Die Parabel, die sich in jedem die Achse 2 enthaltenden Schnitts des Reflektors 3 ergibt, ist durch die folgende Gleichung gegeben:

$$Y^2 = 2px$$

wobei p/2 der Abstand des Brennpunktes F vom Koordinatenursprung ist; die Lage des verwendeten Koordinatensystems x,y ist der Figur zu entnehmen.

Innerhalb der zylindrischen Abstrahlfläche 1 der Schallerzeugungseinheit befindet sich eine Ultraschallortungseinrichtung 11, z. B. in Form eines Ultraschall-B-Bild-Geräts, die zur Darstellung der Fokuszone F im Körper eines Lebewesens 10 dient.

Für die Einkoppelung akustischer Wellen in Körper von Lebewesen ist es in bekannter Weise vorteilhaft, die Wellen bereits in einem Medium mit gewebeähnlichen akustischen Eigenschaften zu erzeugen, um die Reflexionsverluste gering zu halten. Derartige Medien sind beispielsweise Wasser, Öle oder andere Flüssigkeiten, deren akustischen Impedanzen δ * c (Dichte * Schallgeschwindigkeit) der Impedanz von lebendem Gewebe im Einkoppelbereich der Therapiewelle nahekommt. Deshalb ist der Reflektor 3 von einer schalldurchlässigen, akustisch angepaßten Membran 9 abgeschlossen. In dem von dem Reflektor 3 und der Membran 9 gebildeten Raum befindet sich eine Koppelflüssigkeit, so daß sich ein flexibles Kissen ergibt, das unter Vermeidung von Gaseinschlüssen akustisch an den Körper 10 angekoppelt wird.

Die Therapiewellen 4 breiten sich - wie bereits beschrieben - zunächst radial bezüglich der Rotationsachse 2 des Systems aus, und werden anschließend vom Reflektor 3 zum Fokuspunkt F reflektiert. Der akustische Reflektor besitzt dabei die vorstehend beschriebene Form und besteht aus einem Material mit einem hohen Reflexionsgrad in Bezug auf die Koppelflüssigkeit 8.

Das akustische fokussierte Wellenfeld 7 wird über die Membran 9 in den Körper 10 eingekoppelt und mit Hilfe der Ultraschall-Ortungseinheit 11 auf das therapeutische Zielgebiet 12 ausgerichtet. Die Ultraschallsonde ist zur Optimierung der Bildqualität des Ultraschallbildes längs der Achse 2 verschiebbar angeordnet, so daß sie wahlweise bis an den Körper herangeschoben oder aber zur Vermeidung von Abschattungen des Therapiewellenfeldes zurückgezogen werden kann. Ein entsprechender Positionsmelder sorgt dafür, daß der Fokuspunkt des Therapiefeldes kontinuierlich im Ultraschallbild dargestellt wird. Weiterhin ist die Ultraschallsonde um die Achse 2 drehbar angeordnet, so daß das Ultraschallbild wahlweise verschiedene Schnittebene zur Darstellung bringt.

Die Therapieeinheit besitzt neben den notwen-digen Versorgungsanschlüssen für die Therapie-quelle und die Ultraschallsonde hier nicht näher interessierende Anschlüsse für einen Volumenausgleich des Koppelkissens durch Zu-bzw. Ableitung geeignet präparierter Koppelflüssigkeiten, so daß eine akustisch günstige Anlegung der Membran 9 an den Körper 10 möglich wird. Die entsprechen-den Versorgungsgeräte wie Pumpen, Steuereinhei-ten, Entgasungsvorrichtungen usw. sind nicht dargestellt.

Weiterhin ist ohne Beschränkung des allgemei-nen Erfindungsgedankens die Schallerzeugungs-einheit 1 exemplarisch als elektromagnetisches System dargestellt, das innerhalb der Abstrahlfläche 1 eine Spule aufweist. Es können selbstverständlich zur Schallerzeugung auch andere Elemente, bei-spielsweise piezoelektrische Quellen in Zylinder-form verwendet werden.

Der Erfindung geht nun von dem Grundgedanken aus, daß bei einer vorstehend beschriebenen Vorrichtung die Fokussierung des Wellenfeldes "beugungsbegrenzt" ist. Damit ist gemeint, daß der Einfluß der Wellenlänge des Wellenfeldes, das durch die Formgebung der Abstrahlfläche 6 und/oder reflektierende und/oder beugende Elemente 3 "abgebildet" und insbesondere fokussiert wird, vergleichsweise groß ist.

Anders ausgedrückt, kann die Größe des Fokus F in gewissen Grenzen bei ansonsten gleicher Geometrie der das Wellenfeld beeinflußenden Elemente, also insbesondere der Elemente 1 und 3 dadurch variiert werden, daß die Wellenlänge des abgestrahlten Wellenfeldes 4 variiert wird. Die in technischem Maßstab problemlos darstellbaren Wellenlängen-Änderungen sind dabei hinreichend, um die Fokusgröße an medizinische Forderungen anzupaßen, die sich beispielsweise durch die Größe, Art und/oder Lage des zu zertrümmernden Konkrements 12 ergeben.

Bei der in der Figur dargestellten Vorrichtung kann die Schallerzeugungseinheit insbesondere mittels einer nicht dargestellten Entladeeinheit ansteuerbar ist, die eine Kondensator-Bank zur Energiespeicherung aufweist.

Zur Variation der Wellenlänge des Schallfeldes 4 können beispielsweise die einzelnen Kondensatoren der Kondensator-Bank wahlweise parallel und/oder in Reihe schaltbar sein. Ferner ist es auch möglich. lastseitig, d.h. auf der Seite der Spule Kondensatoren und/oder Induktivitäten zuzuschalten.

Besonders vorteilhaft ist es jedoch, wenn zur Variation der Frequenz lastseitig die Induktivität schaltbar ist. Durch die vergleichsweise große Oberfläche der Abstrahlfläche der Schallerzeugungseinheit ist es möglich, daß die Schallerzeugungseinheit mehrere ineinander gewickelte Spulen 1$'$ und 1$''$ aufweist, die alternativ oder miteinander verschaltet vom Strom der (nicht dargestellten) Entladeeinheit durchflossen werden.

Dies bedeutet, daß durch einen externen Um-schaltvorgang, der beispielsweise vom behandeln-den Arzt oder von einer Steuereinheit ausgelöst werden kann, die einzelnen Spulen 1$'$ und 1$''$, die in der Schallerzeugungseinheit 1 angeordnet sind, alternativ oder in einer (teilweisen) Reihen- und/oder Parallelschaltung von der Entladeeinheit angesteuert werden. Da die verschiedenen Spulenkonfigurationen unterschiedliche Induktivitäten aufweisen, ändern sich die Parameter des von den Spulen und den Kondensatoren der Kondensator-Bank zur Energiespeicherung gebildeten Schwingkreises, und damit auch die Wellenlänge des erzeugten Druckwellenfeldes 4.

Die in den vorstehend beschriebenen Maßnahmen haben den Vorteil, daß es durch sie möglich ist, während eines Behandlungsvorgangs die Fokusgröße an die Charakteristiken des zu zerstörenden Konkrements 12, das beispielsweise mittels der Ortungseinheit 11 erfaßt wird, anzupassen. Das Umschalten kann dabei mittels "Knopfdruck" durch den behandelnden Arzt, aber auch durch eine nicht dargestellte Steuereinheit, an die beispielsweise die Signale der Ortungseinheit 11 angelegt sind, erfolgen.

Selbstverständlich ist es aber auch möglich, die Vorrichtung zur Erzeugung von fokussierten akustischen Wellenfeldern für therapeutische Anwendungen und insbesondere zur Zerstörung von Konkrementen, Körpersteinen etc. derart auszugestalten, daß die Schallerzeugungseinheit 1 bzw. die Spulen 1$'$ und 1$''$ austauschbar sind. Dabei ist es bevorzugt, daß sich die austauschbaren Spulen hinsichtlich Durchmesser, Windungszahl und Material der Membran 6 unterscheiden.

Ausdrücklich soll darauf hingewiesen werden, daß bei sehr hohen Amplituden der akustischen Therapiewellen deren Ausbreitungsverhalten vom linearen Ausbreitungsverhalten abweichen kann, so daß sich Veränderungen der Fokusgeometrie ergeben können; diese Veränderungen der Fokusgeometrie können durch entsprechende Anpassung der Reflektorfläche, beispielsweise durch geringfügige Abweichungen von der Parabelform kompensiert werden.

Ebenso können gezielte Änderungen des fokussierten Therapiewellenfeldes durch definierte Abweichungen von der Parabelform und/oder durch eine entsprechende Änderung des Reflexionsverhaltens der Oberfläche erreicht werden.

Es versteht sich von selbst, daß innerhalb des erfindungsgemäßen Grundgedankens - durch eine Änderung der Wellenlänge des Wellenfeldes die "Korrekurbedingungen der akustooptischen Abbildung und damit die Fokusgröße zu variieren - die verschiedensten Modifikationen möglich sind: So ist es beispielsweise möglich, einzelne Maßnahmen beispielsweise zur Variation der Schwingkreisparameter und damit der Wellenlänge miteinander zu kombinieren, und zusätzlich auch austauschbare Spulen vorzusehen.

Ferner ist es natürlich möglich, daß zusätzlich auch die Anstiegszeit variierbar ist, wie dies beispielsweise in dem deutschen Gebrauchsmuster 87 09 692 beschrieben ist.

**Ansprüche**

1. Vorrichtung zur Erzeugung von fokussierten akustischen Wellenfeldern für therapeutische Anwendungen und insbesondere zur Zerstörung von Konkrementen, Körpersteinen etc. mit einer Schallerzeugungseinheit, die in einem Koppelmedium ein Wellenfeld erzeugt, das durch die Formgebung der Abstrahlfläche und/oder das Wellenfeld reflektierende und/oder beugende Elemente fokussiert wird,
dadurch **gekennzeichnet**, daß zur Variation der Fokusgröße die Wellenlänge der abgestrahlten Schallwellen variierbar sind.

2. Vorrichtung nach Anspruch 1, bei der die Schallerzeugungseinheit mittels einer Entladeeinheit ansteuerbar ist, die eine Kondensator-Bank zur Energiespeicherung aufweist,
dadurch **gekennzeichnet**, daß zur Variation der Wellenlänge die einzelnen Kondensatoren der Kondensator-Bank wahlweise parallel und/oder in Reihe schaltbar sind.

3. Vorrichtung nach Anspruch 1 oder 2, bei der die Schallerzeugungseinheit mittels einer Entladeeinheit ansteuerbar ist, die eine Kondensator-Bank zur Energiespeicherung aufweist,

dadurch **gekennzeichnet**, daß zur Variation der Wellenlänge lastseitig Kondensatoren und/oder Induktivitäten zuschaltbar sind.

4. Vorrichtung nach einem der Ansprüche 1 bis 3, bei der die Schallerzeugungseinheit mittels einer Entladeeinheit ansteuerbar ist, die eine Kondensator-Bank zur Energiespeicherung aufweist,
dadurch **gekennzeichnet**, daß zur Variation der Frequenz lastseitig die Induktivität schaltbar sind.

5. Vorrichtung nach einem der Ansprüche 1 bis 4, bei der die Schallerzeugungseinheit als elektromagnetische Druckwellenerzeugungseinheit ausgebildet ist,
dadurch **gekennzeichnet**, daß die Spule austauschbar ist.

6. Vorrichtung nach Anspruch 5,
dadurch **gekennzeichnet**, daß sich die austauschbaren Spulen hinsichtlich Durchmesser, Windungszahl und Membranmaterial unterscheiden.

7. Vorrichtung nach einem der Ansprüche 1 bis 6, bei der die Schallerzeugungseinheit als elektromagnetische Druckwellenerzeugungseinheit ausgebildet ist, die Zylindergeometrie im allgemeinen Sinne aufweist, und mittels einer Entladeeinheit ansteuerbar ist, die eine Kondensator-Bank zur Energiespeicherung aufweist, und deren Wellenfeld ein insbesondere als Paraboloid ausgebildeter Reflektor fokussiert,
dadurch **gekennzeichnet**, daß die Druckwellenerzeugungseinheit mehrere ineinander gewickelte Spulen aufweist, die alternativ oder miteinander verschaltet vom Strom der Entladeeinheit durchflossen werden.

8. Vorrichtung nach einem der Ansprüche 1 bis 7,
dadurch **gekennzeichnet**, daß zusätzlich die Anstiegszeit variierbar ist.

EP 0 387 858 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 254 104 (SIEMENS)(27-01-1988) * Spalte 4, Zeilen 34-55; Spalte 5, Zeile 55 - Spalte 6, Zeile 3; Spalte 6, Zeilen 21-26; Figuren 1,2 * | 1,2,5 | A 61 B 17/22 |
| Y | | 3,4,6-8 | |
| Y | DE-A- 827 418 (FRÜNGEL)(06-12-1951) * Seite 2, Zeilen 9-23; Figur 3 * | 3,4 | |
| Y | US-A-2 964 730 (BLANCHARD)(13-12-1960) * Spalte 1, Zeile 63 - Spalte 2, Zeile 37; Figur 1 * | 6,7 | |
| Y,D | DE-U-8 709 692 (SIEMENS)(05-11-1987) * Seite 3, Zeile 25 - Seite 4, Zeile 3 * | 8 | |

RECHERCHIERTE SACHGEBIETE (Int. Cl.5)

A 61 B
G 10 K

Der vorliegende Recherchenbericht wurde für alle Patentansprüche erstellt

| Recherchenort | Abschlußdatum der Recherche | Prüfer |
|---|---|---|
| DEN HAAG | 07-06-1990 | MOERS R.J. |